# EUROPEAN PATENT SPECIFICATION

(11) **EP 2 007 323 B1**
(45) Date of publication and mention of the grant of the patent: **04.01.2017**
(21) Application number: 07760642.4
(22) Date of filing: 13.04.2007
(51) Int. Cl.: A61B 17/70

(54) **PEDICLE SCREW WITH VERTICAL ADJUSTMENT**
PEDIKELSCHRAUBE MIT VERTIKALER JUSTIERUNG
VIS DE PÉDICULE À RÉGLAGE VERTICAL

(30) Priority: 18.04.2006 US 406134
(43) Date of publication of application: 31.12.2008
(73) Proprietor: International Spinal Innovations, LLC, West Hartford, CT 06107 (US)
(72) Inventor: Aferzon, Joseph, Avon, CT 06001 (US)
(74) Representative: Rasch, Michael
(86) International application number: PCT/US2007/066626
(87) International publication number: WO 2007/121349

(56) References cited:
- WO-A1-2006/005198
- WO-A2-2005/122965
- DE-U1- 20 207 851
- US-A1- 2004 097 933
- US-B1- 6 248 105
- US-B1- 6 478 798
- US-B1- 6 478 798

## Description

This invention relates to a connector to secure a vertebral anchor to a stabilizing rod according to the preamble of claim 1. Preferred embodiments of the invention are set forth in the dependent claims.

Spinal fusion is utilized to correct painful movement across degenerated or unstable spinal segment and is supplemented by fixation with screws. Screws are commonly placed through the posterior vertebral arch. Multiple fixation targets have been used in the past, including facets, spinal processes, lamina, transverse processes, lateral masses and pedicles. Currently, fixation through the pedicles offers the most robust purchase and is utilized when possible.

Screws are implanted from the back to the front of the vertebra with one screw inserted for each side of the vertebra. Individual screws have to be rigidly linked to eliminate movement between corresponding vertebras. The anatomy of an individual patient makes it difficult and often impossible to line up pedicle screws in one plane. Pedicle screws project with variable medial-lateral displacements and different angulations. This places significant challenges on the mechanical design of a linking system that links the pedicle screws. These complex mechanisms with multiple joints have to be manipulated in a deep wound restricted by muscle and soft tissue.

Most of the available systems can be broken into one of three groups: a plating system; a top loading rod system; and a side loading rod system.

The top loading rod system utilizes a seat for the rod that projects along the screw trajectory and forms a ball and socket joint with the spherical screw head. It offers several degrees of freedom about the screw axis: (i) 30-40 degrees of angular deflection from the screw axis; and (ii) 360 degrees of rotation about the screw axis. Any further adjustments have to be gained by contouring the rod to fit into the rod receptacle of the seat. These systems offer advantages when the exposure is directly over the screw such as in muscle splitting or minimally invasive approaches.
In a standard midline approach screws project out into the soft tissue at the lateral extreme of the exposure. The top loading rod system requires manipulation at this lateral extreme and side loading rod system can offer advantages in these circumstances. In the side loading rod system, connectors run perpendicular to the screw trajectory and the rod is placed on the side of the screw toward the center of exposure.

WO 2006/005198 A1 discloses a connector according to the preamble of claim 1. In this arrangement the deflection angle of the anchor with respect to the main axis is limited, i.e., considerably less than 90°. Furthermore, the rod receiver does not allow "vertical" movement of the anchor in relation to the main axis of the connector. This results in restrictions in the use of the connector by the surgeons.
US 6 248 105 discloses a device for connecting a longitudinal rod with a vertebral anchor. The device includes a hollow sleeve with an open channel at the upper end to receive the rod, and with a ring-shaped channel at the lower end to receive a spring chuck. The head of the anchor is snapped into the spring chuck and the spring chuck is radially tightened to secure the head inside the device.

US 2004/0097933 discloses a device for connecting a longitudinal rod with a vertebral anchor. The device includes a connection element with branches at the upper end that form a U-shape to receive the rod, and a bore extending from the U-shape to the lower end at which an internal thread is provided. The head of the anchor is received though the lower end and secured inside the device by a socket having an external thread that is threaded into the internal thread of the device.

WO 2005/122965 discloses a device for connecting a longitudinal rod with a vertebral anchor. The device includes a receiver having upper wall portions extending from a base that form a U-shape at the upper end of the device to receive the rod, and a collar with a threaded central opening below the base to receive the head (post) of the anchor at the lower end of the device. The post of the anchor is secured inside the device by a collet and a set screw that is threaded into the collar.

US 2006/0155277 discloses a device for connecting a longitudinal rod with a vertebral anchor. The device includes coaxial cross-pieces at the upper end that form a U-shape to receive the, and a mount integral with the device encompasses an intermediate element at the lower end of the device. The head of the anchor is received into the intermediate element and secured inside the device by the intermediate element when force is applied by a securing element (nut) threaded into the U-shape.

US 6 478 798 discloses a device for connecting a longitudinal rod with a vertebral anchor. The device includes a clamp, a screw and a swing bolt. The clamp includes two reciprocal clamp elements to secure the rod. The swing bolt is secured to the clamp at one end and pivotally secured to the head of the screw at the opposite end, allowing the screw to pivot with respect to the screw and the clamp.

It is thus object of the invention to avoid these drawbacks and to allow greater angular deflection while still matching full 360° rotation and further allow substantial travel along the screw trajectory contributing to additional degrees of freedom.

These objects are solved by the characterizing features of claim 1.

In accordance with an embodiment, a connector to secure a vertebral anchor to a stabilizing rod is disclosed. The connector includes an anchor clamp that includes a plurality of receptacles to receive a connecting head end of the vertebral anchor secured to a vertebra and to provide angular deflection and rotation of the anchor clamp with respect to the secured vertebral anchor. The connector further includes a compression spacer that includes a top, a tube and a tapered end. The tapered end is to receive at least a portion of the anchor clamp within the tube. The tube is to provide vertical travel and rotation of the anchor clamp with respect to the tube. The connector also includes a rod receiver to house the compression spacer. The rod receiver includes a first end that has a transverse channel to receive the stabilizing rod in the channel and threaded walls to receive a tightening nut. The rod receiver further includes a second end that has a taper to receive the anchor clamp within the compression spacer housed in the rod receiver.

In accordance with another embodiment, an articulation device for fixation of a vertebra to a stabilizing rod is disclosed. The device includes a vertebral anchor that has a first end to engage the vertebra and a connecting head end, and a connector to secure the vertebral anchor to the stabilizing rod. The connector includes an anchor clamp including a plurality of receptacles to receive the connecting head end of the vertebral anchor and to provide angular deflection and rotation of the anchor clamp with respect to the vertebral anchor. The connector further includes a compression spacer including a top, a tube and a tapered end. The tapered end is to receive at least a portion of the anchor clamp within the tube. The tube is to provide vertical displacement and rotation of the anchor clamp with respect to the tube. The connector also includes a rod receiver to house the compression spacer. The rod receiver includes a first end having a transverse channel to receive the stabilizing rod in the channel and the channel having threaded walls to receive a tightening nut. The rod receiver further includes a second end having a taper to receive the anchor clamp within the compression spacer housed in the rod receiver. The threading of the tightening nut within the threaded walls causes the stabilizing rod to displace the compression spacer into the taper of the second end of the rod receiver and to secure the compression spacer within the rod receiver. The displacement of the compression spacer causes a compressive force on the anchor clamp that deforms the plurality of receptacles about the anchor to secure the anchor within the anchor clamp and to secure the anchor clamp within the compression spacer.

In accordance with a further embodiment, an assembly to secure a spinal segment including a plurality of vertebrae is disclosed. The assembly includes at least one stabilizing rod, a plurality of vertebral anchors, each vertebral anchor having a first end to engage a vertebra and a connecting head end, and a plurality of connectors to secure the plurality of vertebral anchors to the at least one stabilizing rod. Each connector includes an anchor clamp including a plurality of receptacles to receive the connecting head end of a vertebral anchor and to provide angular deflection and rotation of the anchor clamp with respect to the vertebral anchor. Each connector further includes a compression spacer including a top, a tube and a tapered end. The tapered end is to receive at least a portion of the anchor clamp within the tube. The tube is to provide vertical travel and rotation of the anchor clamp with respect to the tube. Each connector also includes a rod receiver to house the compression spacer. The rod receiver includes a first end having a transverse channel to receive the at least one stabilizing rod in the channel and threaded walls to receive a tightening nut. The rod receiver further includes a second end having a taper to receive the anchor clamp within the compression spacer.

The following significant advantages are obtained over existing art.
1. Screw-rod articulation functions as a rotating and pivoting joint and not a ball and socket joint characteristic of the top loading systems. It allows much greater angular deflection (over 90 degrees) while still matching full 360-degree rotation.
2. Clamping mechanism allows substantial vertical travel along the screw trajectory contributing to additional degrees of freedom. This adds substantial flexibility particularly when more than three (3) screws need to be connected.
3. Connector can be snapped onto the screw head after screw is placed into the bone. This keeps hardware profile minimal and improves exposure for delivery of graft material. It also allows for all the connectors and rods to be assembled outside of the wound. This is particularly valuable in small direct exposures characteristic of the minimally invasive approach.
4. Can be utilized both as a top loading and side loading system, which can not be matched by the currently available art.

### Description

In a preferred embodiment device consists of a screw, screw clamp, rod receiver, compression spacer, rod and nut (Fig.1). The screw has a long threaded portion placed into the bone and a protruding cylindrical shaft with a spherical head (Fig. 2). A compressible cylindrically shaped screw clamp of substantial thickness (Fig. 3) has a spherical cut-out at one end such that it fits precisely over the screw head (Fig. 4). The screw clamp pivots about the screw head with angle of deflection greater then ninety degrees and 360 degrees of revolution through the entire range of deflection.

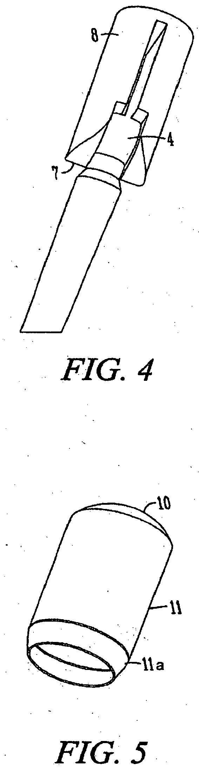

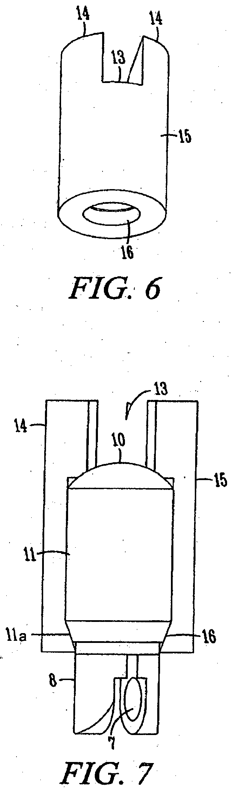

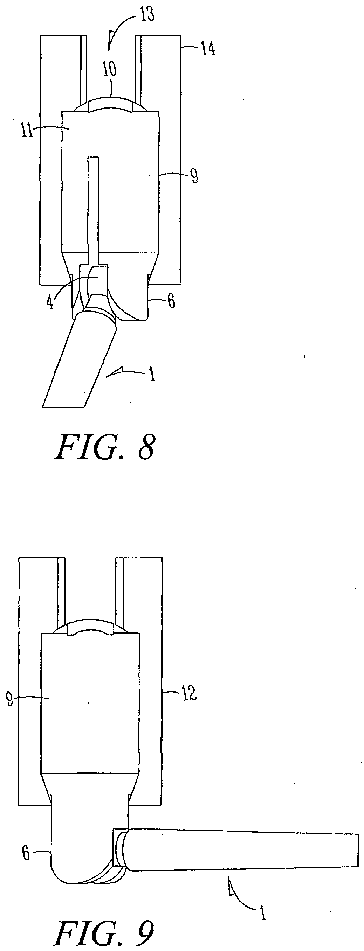

The screw clamp (Fig. 6) fits tightly into a cylindrical assembly consisting of the rod receiver and the compression spacer (Fig. 7). The rod receiver has a travel tube with a tapered end, which fits over the screw clamp. The other end of the rod receiver forms the seat for the rod bounded by threaded walls (Fig. 8). Up and down movement of the screw clamp within the rod receiver allows for a vertical adjustment. The compression clamp is a deformable component fitting within the travel tube of the rod receiver (Fig. 5). The compression clamp has a contact dome extending into the seat of the rod receiver. Pressure from the rod locked into the seat by a threaded nut displaces the compression spacer into the tapered end of the travel tube. The resulting deformation of the compression spacer transfers compressive force onto the screw clamp. This fixes the screw head within the screw clamp and prevents any travel of the screw clamp within the travel tube. This process is repeated for each screw and cylindrical assembly.

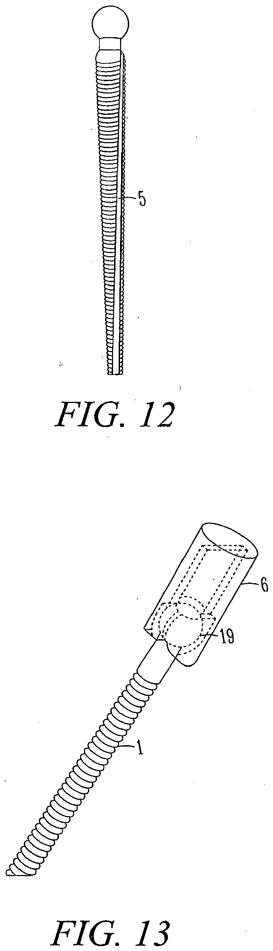

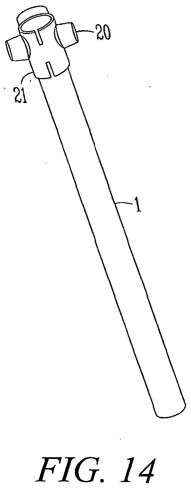

Alternative embodiments of the screw shaft and screw clamp articulation which enable pivoting and rotational motion are possible. (Fig. 13, Fig. 14).

### Brief description of the drawings

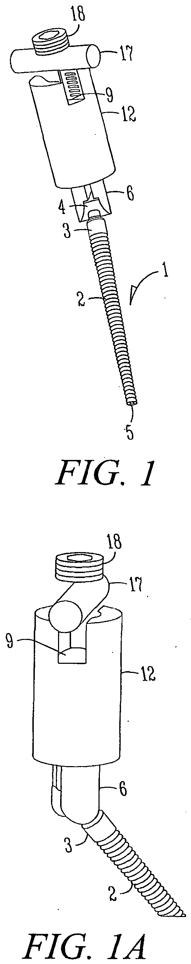

Figs. 1-14 include the following components:

| | |
|---|---|
| Screw | (1) |
| Threaded portion | (2) |
| Protruding shaft | (3) |
| Spherical head | (4) |
| Central channel | (5) |
| Screw clamp | (6) |
| Articulating end | (7) |
| Compressible shaft | (8) |
| Compression Spacer | (9) |
| Contact Dome | (10) |
| Constricting Tube | (11) |
| Rod receiver | (12) |
| Seat | (13) |
| Threaded extensions | (14) |
| Travel tube | (15) |
| Tapered end | (16) |
| Rod | (17) |
| Nut | (18) |
| Spherical spacer | (19) |
| T-spacer | (21) |
| Projections | (20) |

Fig. 1 illustrates a particular embodiment of an assembly that includes a screw (1), a screw clamp (6), a compression spacer (9), a rod receiver 12, a rod (17) and a nut (18);
Fig. la illustrates a different orientation of the particular embodiment showing the screw (1), the screw clamp (6), the compression spacer (9), the rod receiver 12, the rod (17) and the nut (18);

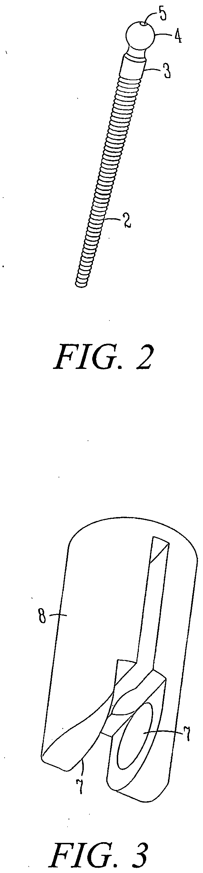

Fig. 2 illustrates the bone screw (1) consisting of a threaded portion (2), a protruding shaft (3) and a spherical head (4), and a channel (5) through a center of the screw (1);
Fig. 3 illustrates a particular embodiment of the screw clamp (6) consisting of a cylindrical compressible shaft (8) and articulating ends (7);
Fig. 4 illustrates pivoting articulation between the screw head (4) and articulating ends (7) of the screw clamp (6) ;
Fig. 5 illustrates an embodiment of the compression spacer (9) with a contact dome (10) that is depressed by the rod (17) when it is locked, and a constricting tube (11) deformed by a tapered section (16) of the rod receiver (12) illustrated in Fig. 6;
Fig. 6 illustrates a particular embodiment of the rod receiver (12) consisting of a seat (13) for the rod (17), threaded extensions (14) that accept a nut (18), a travel tube (15) and a tapered end (16);
Fig. 7 illustrates an assembly that consists of the screw clamp (6) having the compressible shaft (8) and the articulating ends (7), a compression spacer (9) having the contact dome (10) and the constricting tube (11), and the rod receiver (12);
Fig. 8 illustrates a particular embodiment with the compression spacer (9) and the rod receiver (12) rendered transparent to demonstrate the deformable compression spacer (9) being pressed into the tapered end (16) of the rod receiver (12) that constricts the compression spacer (9) and the compression spacer (9) in turn transmits compressive forces to the articulation between screw clamp (6) and a screw (1);
Fig. 9 illustrates at least a 90-degree deflection angle between the screw (1) and the screw clamp (6);

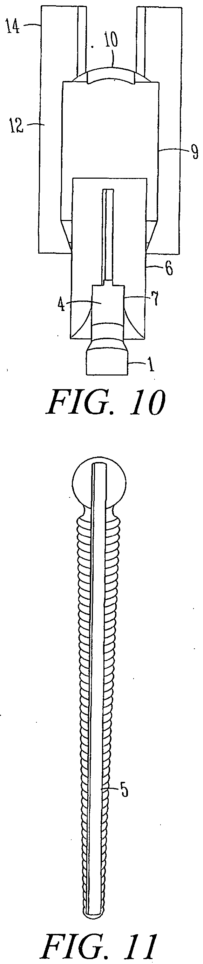

Fig. 10 illustrates vertical travel of the screw clamp (6) within the compression spacer (9) of the rod receiver (12)-;
Fig. 11 illustrates the screw (1) with a central channel (5)-;
Fig. 12. illustrates the screw (1) with center channel (5) that is off center-;
Fig. 13 illustrates an embodiment of the screw (1) and the screw clamp (6) articulation, in which a compressible spherical spacer (19) threaded over the cylindrical screw shaft (3)-; and
Fig. 14 illustrates another embodiment of the screw (1) and screw clamp (6) articulation, in which the screw clamp (6) is modified to fit over projections (20) of a compressible T-spacer (21) of the screw (1).

## Claims

1. A connector to secure a vertebral anchor (1) with a connecting head end (4) to a stabilizing rod (17), the connector comprising:
a screw clamp (6) to receive the connecting head end (4) and to provide angular deflection and rotation of the vertebral anchor (1) with respect to the screw clamp (6);
a compression spacer (9) including a dome (10) to receive the screw clamp (6) within the compression spacer (9) to provide rotation of the screw clamp (6) with respect to the compression spacer (9); and
a rod receiver (12) to house the compression spacer (9), the rod receiver (12) including a first end having a transverse channel (13) to receive the stabilizing rod (17) in the transverse channel (13) and threaded extensions (14) to receive a tightening nut (18), **characterized in that**:
the screw clamp (6) comprises a cylindrical compressible shaft (8) and an articulating end (7), the articulating end (7) to receive the connecting head end (4);
the compression spacer (9) comprises a constricting tube (11) having an external tapered end (11a), the constricting tube (11) receiving a portion of the screw clamp (6) within the constricting tube (11) through the external tapered end (11a), the constricting tube (11) providing vertical travel and rotation to the cylindrical compressible shaft (8) of the screw clamp (6) with respect to the compression spacer (9); and
the rod receiver (12) comprises a second end having an internal tapered section (16) that cooperates with the external tapered end (11a) of the constricting tube (11) (i) in a first vertical abutting position in which the screw clamp (6) is received partially within the compression spacer (9) allowing vertical travel and rotation to the screw clamp (6) with respect to the compression spacer (9) with the articulating end (7) of the screw clamp (6) being disposed outside the rod receiver (12), and (ii) in a second vertical abutting position in which the constricting tube (11) is compressed about the screw clamp (6) securing the connecting head end (4) of the vertebral anchor (1) with respect to the articulating end (7) of the screw clamp (6) outside the rod receiver (12).

2. The connector of claim 1, wherein the articulating end (7) of the screw clamp (6) has one of an uneven knurled surfaces and smooth surfaces.

3. The connector of claim 1, wherein a threading of the tightening nut (18) within the threaded walls (14) causes the stabilizing rod (17) to displace the compression spacer (9) with the external tapered end (11a) engaging the internal tapered section (16) of the rod receiver (12) and to secure the compression spacer within the rod receiver, the displacement of the compression spacer causing a compressive force on the screw clamp (6) that deforms the articulating end (7) about the connecting head end (4) of the vertebral anchor to secure the vertebral anchor (1) with respect to the screw clamp (6) and to secure the screw clamp (6) within the compression spacer (9).

4. An assembly to secure a spinal segment including a plurality of vertebrae, the assembly including a plurality of connectors according to claim 1, the assembly comprising:
at least one stabilizing rod (17);
a plurality of vertebral anchors (1), each vertebral anchor having a first end to be secured to a vertebra and a connecting head end (4); and
the plurality of connectors to secure the plurality of vertebral anchors (1) to the at least one stabilizing rod (17).

5. The assembly of claim 4, wherein the at least one stabilizing rod (17) comprises a first stabilizing rod to be disposed on a first side of the spinal segment and a second stabilizing rod to be disposed on a second side of the spinal segment.

6. The assembly of claim 5, further comprising a transverse bridging member to link the first stabilizing rod and the second stabilizing rod.

7. The assembly of claim 4, wherein a vertebral anchor comprises a means for handling the vertebral anchor to engage a vertebra.

8. The assembly of claim 7, wherein the means for handling the vertebral anchor includes one or more protrusions and one or more surface indentations on the vertebral anchor.

9. The assembly of claim 4, wherein the at least one stabilizing rod includes one or more protrusions and one or more surface indentations at each end of the at least one stabilizing rod.

10. The assembly of claim 4, wherein the first end of the vertebral anchor is a screw (1) and the connecting head end (4) of the vertebral anchor is of a substantially spherical shape or a cylindrical shape.

11. The assembly of claim 10, wherein the vertebral anchor includes a channel (5) through a central axis of the screw (1) and the connecting head end (4).

12. The assembly of claim 11, wherein the vertebral anchor includes an off-center channel (5) extending from a tip of the screw through to a side of the screw.

13. The assembly of claim 4, wherein the threaded walls of the channel (5) include extensions to project above a skin surf ace.

14. The assembly of claim 4, wherein the vertebral anchor (1) is one of a lamina hook, a transverse process hook, a facet screw, and a facet bolt.

15. The assembly of claim 4, wherein the connecting head end (4) of the vertebral anchor (1) has one of an uneven knurled surface and a smooth surface.

## Patentansprüche

1. Verbinder zum Sichern eines Wirbelankers (1), der ein Verbindungskopfende (4) aufweist, mit einem Stabilisierungsstab (17), wobei der Verbinder Folgendes umfasst:
eine Schraubzwinge (6) zum Aufnehmen des Verbindungskopfendes (4) und zum Bereitstellen einer Winkelauslenkung und Rotation des Wirbelankers (1) mit Bezug auf die Schraubzwinge (6);
einen Kompressionsabstandshalter (9), der eine Kuppel (10) zum Aufnehmen der Schraubzwinge (6) innerhalb des Kompressionsabstandshalters (9) zum Bereitstellen einer Rotation der Schraubzwinge (6) mit Bezug auf den Kompressionsabstandshalter (9) enthält; und
eine Stabaufnahme (12) zum Aufnehmen des Kompressionsabstandshalters (9), wobei die Stabaufnahme (12) ein erstes Ende enthält, das einen Querkanal (13) zum Aufnehmen des Stabilisierungsstabes (17) in dem Querkanal (13) sowie Gewindevorsprünge (14) zum Aufnehmen einer Festziehmutter (18) aufweist, **dadurch gekennzeichnet, dass**:
die Schraubzwinge (6) eine zylindrische komprimierbare Welle (8) und ein Gelenk-Ende (7) umfasst, wobei das Gelenk-Ende (7) zum Aufnehmen des Verbindungskopfendes (4) dient;
der Kompressionsabstandshalter (9) ein Verengungsrohr (11) umfasst, das ein äußeres verjüngtes Ende (11a) aufweist, wobei das Verengungsrohr (11) einen Abschnitt der Schraubzwinge (6) innerhalb des Verengungsrohres (11) durch das äußere verjüngte Ende (11a) hindurch aufnimmt, wobei das Verengungsrohr (11) vertikalen Vorschub und Rotation für die zylindrische komprimierbare Welle (8) der Schraubzwinge (6) mit Bezug auf den Kompressionsabstandshalter (9) erlaubt; und
die Stabaufnahme (12) ein zweites Ende umfasst, das eine innere verjüngte Sektion (16) aufweist, die mit dem äußeren verjüngten Ende (11a) des Verengungsrohres (11) (i) in einer ersten vertikalen Anliegeposition zusammenwirkt, in der die Schraubzwinge (6) teilweise in dem Kompressionsabstandshalter (9) aufgenommen ist, wodurch vertikaler Vorschub und Rotation für die Schraubzwinge (6) mit Bezug auf den Kompressionsabstandshalter (9) ermöglicht werden, wobei das Gelenk-Ende (7) der Schraubzwinge (6) außerhalb der Stabaufnahme (12) angeordnet ist, und (ii) in einer zweiten vertikalen Anliegeposition zusammenwirkt, in der das Verengungsrohr (11) um die Schraubzwinge (6) herum zusammengedrückt wird, so dass das Verbindungskopfende (4) des Wirbelankers (1) mit Bezug auf das Gelenk-Ende (7) der Schraubzwinge (6) außerhalb der Stabaufnahme (12) gesichert wird.

2. Verbinder nach Anspruch 1, wobei das Gelenk-Ende (7) der Schraubzwinge (6) unebene gerändelte Oberflächen oder glatte Oberflächen aufweist.

3. Verbinder nach Anspruch 1, wobei ein Gewinde der Festziehmutter (18) innerhalb der mit einem Gewinde versehenen Wände (14) bewirkt, dass der Stabilisierungsstab (17) den Kompressionsabstandshalter (9) verschiebt, wobei das äußere verjüngte Ende (11a) die innere verjüngte Sektion (16) der Stabaufnahme (12) in Eingriff nimmt, und den Kompressionsabstandshalter innerhalb der Stabaufnahme sichert, wobei die Verschiebung des Kompressionsabstandshalters eine Kompressionskraft auf die Schraubzwinge (6) ausübt, die das Gelenk-Ende (7) um das Verbindungskopfende (4) des Wirbelankers herum verformt, um den Wirbelanker (1) mit Bezug auf die Schraubzwinge (6) zu sichern und die Schraubzwinge (6) innerhalb des Kompressionsabstandshalters (9) zu sichern.

4. Baugruppe zum Sichern eines Wirbelsäulensegments, das mehrere Wirbel enthält, wobei die Baugruppe mehrere Verbinder nach Anspruch 1 enthält, wobei die Baugruppe Folgendes umfasst:
mindestens einen Stabilisierungsstab (17);
mehrere Wirbelanker (1), wobei jeder Wirbelanker ein erstes Ende, das an einem Wirbel zu sichern ist, und ein Verbindungskopfende (4) aufweist; und
wobei die mehreren Verbinder zum Sichern der mehreren Wirbelanker (1) an dem mindestens einen Stabilisierungsstab (17) dienen.

5. Baugruppe nach Anspruch 4, wobei der mindestens eine Stabilisierungsstab (17) einen ersten Stabilisierungsstab umfasst, der auf einer ersten Seite des Wirbelsäulensegments anzuordnen ist, und einen zweiten Stabilisierungsstab umfasst, der auf einer zweiten Seite des Wirbelsäulensegments anzuordnen ist.

6. Baugruppe nach Anspruch 5, die des Weiteren ein Querbrückenelement umfasst, um den ersten Stabilisierungsstab und den zweiten Stabilisierungsstab zu verbinden.

7. Baugruppe nach Anspruch 4, wobei ein Wirbelanker ein Mittel zum Handhaben des Wirbelankers für eine Eingriffnahme eines Wirbels umfasst.

8. Baugruppe nach Anspruch 7, wobei das Mittel zum Handhaben des Wirbelankers einen oder mehrere Vorsprünge und eine oder mehrere Oberflächenvertiefungen an dem Wirbelanker enthält.

9. Baugruppe nach Anspruch 4, wobei der mindestens eine Stabilisierungsstab einen oder mehrere Vorsprünge und eine oder mehrere Oberflächenvertiefungen an jedem Ende des mindestens einen Stabilisierungsstabes enthält.

10. Baugruppe nach Anspruch 4, wobei das erste Ende des Wirbelankers eine Schraube (1) ist, und das Verbindungskopfende (4) des Wirbelankers eine im Wesentlichen sphärische Form oder eine zylindrische Form hat.

11. Baugruppe nach Anspruch 10, wobei der Wirbelanker einen Kanal (5) durch eine Mittelachse der Schraube (1) und das Verbindungskopfende (4) hindurch enthält.

12. Baugruppe nach Anspruch 11, wobei der Wirbelanker einen außermittigen Kanal (5) enthält, der sich von einer Spitze der Schraube bis zu eine Seite der Schraube erstreckt.

13. Baugruppe nach Anspruch 4, wobei die mit einem Gewinde versehenen Wände des Kanals (5) Verlängerungen enthalten, die über die Hautoberfläche hinausragen.

14. Baugruppe nach Anspruch 4, wobei der Wirbelanker (1) ein Laminahaken, ein Querfortsatzhaken, eine Facettenschraube oder ein Facettenbolzen ist.

15. Baugruppe nach Anspruch 4, wobei das Verbindungskopfende (4) des Wirbelankers (1) eine unebene gerändelte Oberfläche oder eine glatte Oberfläche aufweist.

## Revendications

1. Connecteur pour fixer un ancrage vertébral (1) avec une tête de raccordement (4) sur une tige de stabilisation (17), le connecteur comprenant :
un dispositif de serrage (6) destiné à recevoir la tête de raccordement (4) et à fournir une déflection angulaire et une rotation de l'ancrage vertébral (1) par rapport au dispositif de serrage (6) ;
un dispositif d'espacement par compression (9) incluant un dôme (10) destiné à recevoir le dispositif de serrage (6) à l'intérieur du dispositif d'espacement par compression (9) pour fournir une rotation du dispositif de serrage (6) par rapport au dispositif d'espacement par compression (9) ; et
un récepteur de tige (12) destiné à loger le dispositif d'espacement par compression (9), le récepteur de tige (12) comprenant une première extrémité ayant un canal transversal (13) destiné à recevoir la tige de stabilisation (17) dans le canal transversal (13) et des extensions filetées (14) destinées à recevoir un écrou de serrage (18), **caractérisé en ce que** :
le dispositif de serrage (6) comprend un arbre compressible cylindrique (8) et une extrémité d'articulation (7), l'extrémité d'articulation (7) étant destinée à recevoir la tête de raccordement (4) ;
le dispositif d'espacement par compression (9) comprend un tube de constriction (11) ayant une extrémité filetée externe (11a), le tube de constriction (11) recevant une partie du dispositif de serrage (6) à l'intérieur du tube de constriction (11) par le biais de l'extrémité filetée externe (11a), le tube de constriction (11) fournissant un déplacement vertical et une rotation à l'arbre compressible cylindrique (8) du dispositif de serrage (6) par rapport au dispositif d'espacement par compression (9) ; et
le récepteur de tige (12) comprend une seconde extrémité ayant une section filetée interne (16) qui coopère avec l'extrémité fileté externe (11a) du tube de constriction (11) (i) dans une première position contiguë verticale dans laquelle le dispositif de serrage (6) est partiellement réceptionné à l'intérieur du dispositif d'espacement par compression (9), permettant un déplacement vertical et une rotation du dispositif de serrage (6) par rapport au dispositif d'espacement par compression (9), l'extrémité d'articulation (7) du dispositif de serrage (6) étant placée à l'extérieur du récepteur de tige (12), et (ii) dans une seconde position contiguë verticale dans laquelle le tube de constriction (11) est comprimé autour du dispositif de serrage (6), fixant la tête de raccordement (4) de l'ancrage vertébral (1) par rapport à l'extrémité d'articulation (7) du dispositif de serrage (6) à l'extérieur du récepteur de tige (12).

2. Connecteur selon la revendication 1, dans lequel l'extrémité d'articulation (7) du dispositif de serrage (6) a des surfaces moletées irrégulières ou des surfaces lisses.

3. Connecteur selon la revendication 1, dans lequel un filetage de l'écrou de serrage (18) à l'intérieur des parois filetées (14) provoque le déplacement par la tige de stabilisation (17) du dispositif d'espacement par compression (9), l'extrémité filetée externe (11a) engageant la section filetée interne (16) du récepteur de tige (12) et la fixation du dispositif d'espacement par compression à l'intérieur du récepteur de tige, le déplacement du dispositif d'espacement par compression provoquant une force compressive sur le dispositif de serrage (6) qui déforme l'extrémité d'articulation (7) autour de la tête de raccordement (4) de l'ancrage vertébral pour fixer l'ancrage vertébral (1) par rapport au dispositif de serrage (6) et pour fixer le dispositif de serrage (6) à l'intérieur du dispositif d'espacement par compression (9).

4. Assemblage pour fixer un segment spinal comprenant une pluralité de vertèbres, l'assemblage comprenant une pluralité de connecteurs selon la revendication 1, l'assemblage comprenant :
au moins une tige de stabilisation (17) ;
une pluralité d'ancrages vertébraux (1), chaque ancrage vertébral ayant une première extrémité devant être fixée sur une vertèbre et une tête de raccordement (4) ; et
la pluralité de connecteurs pour fixer la pluralité d'ancrages vertébraux (1) sur l'au moins une tige de stabilisation (17).

5. Assemblage selon la revendication 4, dans lequel l'au moins une tige de stabilisation (17) comprend une première tige de stabilisation devant être placée sur un premier côté du segment spinal et une seconde tige de stabilisation devant être placée sur un second côté du segment spinal.

6. Assemblage selon la revendication 5, comprenant en outre un élément pontant transversal destiné à lier la première tige de stabilisation et la seconde tige de stabilisation.

7. Assemblage selon la revendication 4, dans lequel un ancrage vertébral comprend un moyen destiné à la manipulation de l'ancrage vertébral pour engager une vertèbre.

8. Assemblage selon la revendication 7, dans lequel le moyen destiné à la manipulation de l'ancrage vertébral comprend une ou plusieurs protrusions et une ou plusieurs indentations de surface sur l'ancrage vertébral.

9. Assemblage selon la revendication 4, dans lequel l'au moins une tige de stabilisation comprend une ou plusieurs protrusions et une ou plusieurs indentations de surface à chaque extrémité de l'au moins une tige de stabilisation.

10. Assemblage selon la revendication 4, dans lequel la première extrémité de l'ancrage vertébral est une vis (1) et la tête de raccordement (4) de l'ancrage vertébral est de forme sensiblement sphérique ou de forme cylindrique.

11. Assemblage selon la revendication 10, dans lequel l'ancrage vertébral comprend un canal (5) à travers un axe central de la vis (1) et la tête de raccordement (4).

12. Assemblage selon la revendication 11, dans lequel l'ancrage vertébral comprend un canal décentré (5) se prolongeant d'une pointe de la vis jusqu'à un côté de la vis.

13. Assemblage selon la revendication 4, dans lequel les parois filetées du canal (5) comprennent des extensions pour se projeter au-dessus d'une surface cutanée.

14. Assemblage selon la revendication 4, dans lequel l'ancrage vertébral (1) est choisi parmi un crochet de lamine, un crochet de procédé transversal, une vis à facette et un boulon à facette.

15. Assemblage selon la revendication 4, dans lequel la tête de raccordement (4) de l'ancrage vertébral (1) a des surfaces moletées irrégulières ou des surfaces lisses.
